# EUROPEAN PATENT APPLICATION

(11) **EP 4 681 716 A1**
(43) Date of publication of application: **21.01.2026**
(21) Application number: 25189230.3
(22) Date of filing: 14.07.2025
(51) Int. Cl.: A61K 31/7032, A61K 36/704, A61P 21/00, A23L 33/105

(54) **COMPOSITION FOR ENHANCING EXERCISE PERFORMANCE, INCREASING MUSCLE MASS, IMPROVING MUSCLE FUNCTIONS, OR PREVENTING OR TREATING MUSCULAR DISEASE CONTAINING POLYGONUM MULTIFLORUM THUNBERG EXTRACT**

(30) Priority: 17.07.2024 KR 20240094370
(71) Applicant: Korea Institute of Science and Technology, Seoul 02792 (KR); UNIVERSITY-INDUSTRY COOPERATION GROUP OF KYUNG HEE UNIVERSITY, Yongin-si, Gyeonggi-do 17104 (KR)
(72) Inventor: Myungsuk, Kim, 25451 Gangwon-do (KR); Jung, Sang Hoon, 25451 Gangwon-do (KR); Hye-Young, Youn, 25451 Gangwon-do (KR); Tam, Thi Le, 25451 Gangwon-do (KR); Pham, Thi Thu Trang, 25451 Gangwon-do (KR); Lim, Seo Yeon, 25451 Gangwon-do (KR); Chang, Joonyeon, 25451 Gangwon-do (KR); Chung, Won Suk, 02722 Seoul (KR); Jung, Hee Jae, 04368 Seoul (KR); Byung, Cheol Lee, 04700 Seoul (KR)
(74) Representative: Handley, Matthew Edward

(57) **Abstract**

The present invention relates to a composition for enhancing exercise performance, increasing muscle mass, improving muscle function, or preventing or treating muscular disease, comprising *Polygonum multiflorum* Thunberg extract. The extract of the present invention has the effect of enhancing muscle strength, increasing muscle mass, improving exercise performance, and the like.

## Description

### [Technical field]

This application claims benefit of priority based on Korean Patent Application No. 10-2024-0094370 filed on July 17, 2024, the disclosures of which are incorporated herein by reference in their entireties.

The present invention relates to a composition for enhancing exercise performance, increasing muscle mass, improving muscle functions, or preventing or treating muscular disease, comprising *Polygonum multiflorum* Thunberg extract.

### [Background art]

Muscle can be categorized into skeletal muscle, cardiac muscle, and visceral muscle, with skeletal muscle being the most abundant tissue in the human body, making up 40-45% of body weight. Skeletal muscles attach to bones via tendons and are responsible for generating bone movement or force. A muscle is made up of many myofibers, which in turn are made up of many myofibrils, which are made up of actin and myosin. When actin and myosin overlap and move, the length of the muscle shortens or lengthens, causing contraction and relaxation of the overall muscle. An increase in the size of the myofibrils means an increase in the thickness of the myofibers, resulting in muscle gain.

Muscular diseases are characterized by a progressive progression of skeletal muscle weakness that leads to impaired walking and mobility, difficulty with activities of daily living (ADLs), and loss of independence. In addition, they can lead to cardiopulmonary dysfunction and other complications, so it is important to accurately understand the characteristics of each muscular disease and approach it accordingly.

South Korea entered an aging society in 2000 with the elderly population accounting for 7.2% of the total population, and is expected to enter a super-aging society (more than 20%) in 2050 (2013 statistics on the elderly, Statistics Korea). As a person's muscle mass decreases with age (by 10-15% between the ages of 50 and 70, and by more than 30% between the ages of 70 and 80), muscle strength and muscle function also decrease, a condition known as sarcopenia. Senile sarcopenia is a major cause that limits the independent living of older people by causing activity and walking difficulties. In addition, sarcopenia lowers basal metabolic rate, increases insulin resistance, promotes type 2 diabetes, and increases the risk of high blood pressure and cardiovascular disease by 3-5 times. Currently, there are no drugs approved for the treatment of sarcopenia, and drug repositioning technology that applies myostatin inhibitors or existing FDA-approved treatments for other diseases to sarcopenia is being developed.

The present inventors have made efforts to develop a novel material and/or a natural product that has effects of enhancing exercise performance, increasing muscle mass, improving muscle functions, or preventing or treating muscular disease, and as a result, have experimentally confirmed that an extract of *Polygonum multiflorum* Thunberg is useful for increasing muscle weight, improving muscle strength, and improving exercise performance, thereby completing the present invention.

### [Disclosure]

### [Technical Problem]

It is an object of the present invention to provide a food composition for enhancing exercise performance, increasing muscle mass, improving muscle function, or preventing or ameliorating muscular disease, comprising *Polygonum multiflorum* Thunberg extract as an active ingredient.

It is also an object of the present invention to provide a quasi-drug composition for improving exercise performance, increasing muscle mass, improving muscle function, or preventing or ameliorating muscular disease, comprising a *Polygonum multiflorum* Thunberg extract as an active ingredient.

It is also an object of the present invention to provide a pharmaceutical composition for enhancing exercise performance, increasing muscle mass, improving muscle function, or preventing or treating muscular disease, comprising *Polygonum multiflorum* Thunberg extract as an active ingredient.

It is also an object of the present invention to provide a food composition for enhancing exercise performance, increasing muscle mass, improving muscle function, or preventing or ameliorating muscular disease, comprising a compound represented by Formula 1 as an active ingredient.

It is also an object of the present invention to provide a quasi-drug composition for improving exercise performance, increasing muscle mass, improving muscle function, or preventing or ameliorating muscular disease, comprising a compound represented by Formula 1 as an active ingredient.

It is also an object of the present invention to provide a pharmaceutical composition for enhancing exercise performance, increasing muscle mass, improving muscle function, or preventing or treating muscular disease, comprising a compound represented by Formula 1 as an active ingredient.

It is also an object of the present invention to provide a use of a composition comprising *Polygonum multiflorum* Thunberg extract or a compound represented by Formula 1 an active ingredient for the manufacture of a medicament/food composition for enhancing exercise performance, increasing muscle mass, improving muscle function, or preventing or treating/ameliorating muscular disease.

It is also an object of the present invention to provide a method for preventing or treating/ameliorating muscular disease, or a method for enhancing exercise performance, increasing muscle mass, or improving exercise performance, comprising administering or taking a composition comprising *Polygonum multiflorum* Thunberg extract or a compound represented by Formula 1 as an active ingredient to an individual in need.

However, the technical problem to be achieved by the present invention is not limited to the problems mentioned above, and other problems not mentioned will be clearly understood by those skilled in the art from the description below.

### [Technical solution]

The present invention provides a food composition for enhancing exercise performance, increasing muscle mass, improving muscle function, or preventing or ameliorating muscular disease, comprising *Polygonum multiflorum* Thunberg extract as an active ingredient.

The present invention also provides a quasi-drug composition for improving exercise performance, increasing muscle mass, improving muscle function, or preventing or ameliorating muscular disease, comprising *Polygonum multiflorum* Thunberg extract as an active ingredient.

The present invention also provides a pharmaceutical composition for enhancing exercise performance, increasing muscle mass, improving muscle function, or preventing or treating muscular disease, comprising *Polygonum multiflorum* Thunberg extract as an active ingredient.

The present invention also provides a use of a composition comprising *Polygonum multiflorum* Thunberg extract or a compound represented by Formula 1 an active ingredient for the manufacture of a medicament/food composition for enhancing exercise performance, increasing muscle mass, improving muscle function, or preventing or treating/ameliorating muscular disease.

The present invention also provides a method for preventing or treating/ameliorating muscular disease, or a method for enhancing exercise performance, increasing muscle mass, or improving exercise performance, comprising administering or taking a composition comprising *Polygonum multiflorum* Thunberg extract as an active ingredient to an individual in need.

The composition may enhance grip and/or muscle strength.

The composition may increase ATP production.

The composition may increase the expression of one or more genes selected from the group consisting of *Myod, Myog, Myh2, Myf5, Myf6, Nfr1,* and *Ucp3.*

The present *Polygonum multiflorum* Thunberg extract may comprise the compound represented by Formula 1 below (2,3,5,4'-Tetrahydroxystilbene-2-O-β-D-glucoside, THSG):

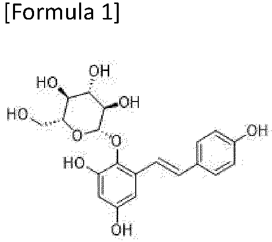

The composition may be extracted with a solvent selected from the group consisting of water, organic solvents, and mixtures thereof.

The organic solvent may be one or more solvents selected from the group consisting of lower alcohols of carbon number 1 to 6, hexane, acetone, ethyl acetate, and diethyl ether.

The muscular disease may be one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

The present invention also provides a food composition for enhancing exercise performance, increasing muscle mass, improving muscle function, or preventing or ameliorating muscular disease, comprising a compound represented by Formula 1 as an active ingredient.

The present invention also provides a quasi-drug composition for improving exercise performance, increasing muscle mass, improving muscle function, or preventing or ameliorating muscular disease, comprising a compound represented by Formula 1 as an active ingredient.

The present invention also provides a pharmaceutical composition for enhancing exercise performance, increasing muscle mass, improving muscle function, or preventing or treating muscular disease, comprising a compound represented by Formula 1 as an active ingredient.

The present invention also provides a use of a composition comprising a compound represented by Formula 1 an active ingredient for the manufacture of a medicament/food composition for enhancing exercise performance, increasing muscle mass, improving muscle function, or preventing or treating/ameliorating muscular disease.

The present invention also provides a method for preventing or treating/ameliorating muscular disease, or a method for enhancing exercise performance, increasing muscle mass, or improving exercise performance, comprising administering or taking a composition comprising a compound represented by Formula 1 as an active ingredient to an individual in need.

### [Advantageous Effects]

The extract of *Polygonum multiflorum* Thunberg of the present invention has the effect of enhancing muscle/grip strength, increasing muscle mass/weight, improving exercise performance, and the like. Furthermore, the present composition comprising the *Polygonum multiflorum* Thunberg extract as an active ingredient can be applied to various products such as pharmaceutical composition, food composition, and quasi-drug composition for improving exercise performance, increasing muscle mass, improving muscle function, or preventing, treating or ameliorating muscular disease as a natural product.

### [Description of Drawings]

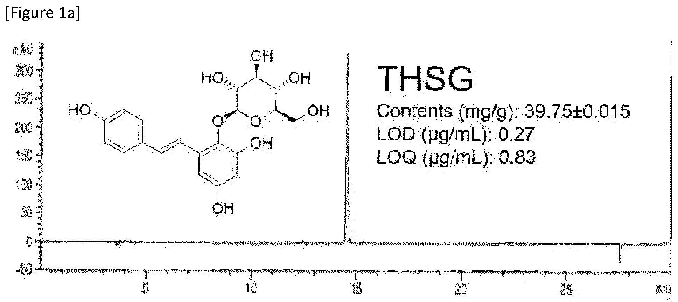

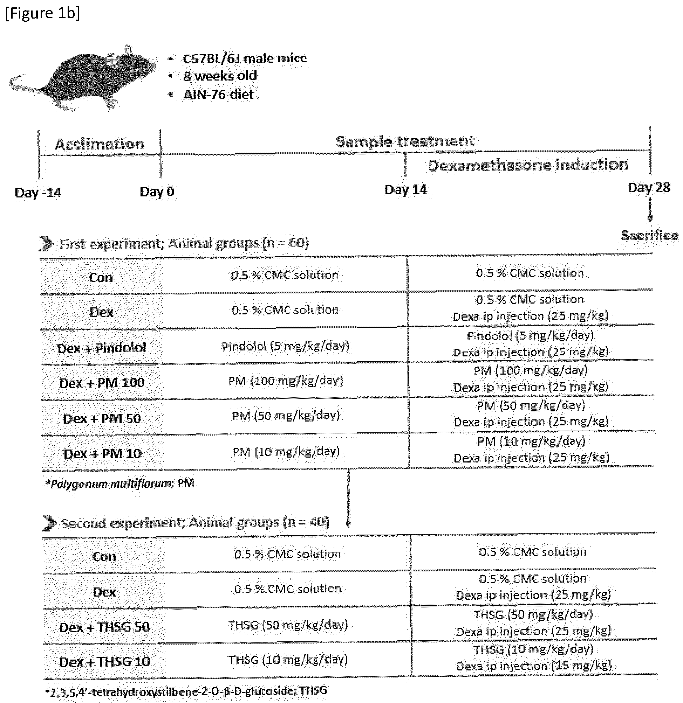

Figure 1a shows the compounds isolated from *Polygonum multiflorum* Thunberg extract. Figure 1b is a schematic diagram of an animal model design to test the effectiveness of *Polygonum multiflorum* Thunberg extract in treating or ameliorating sarcopenia.

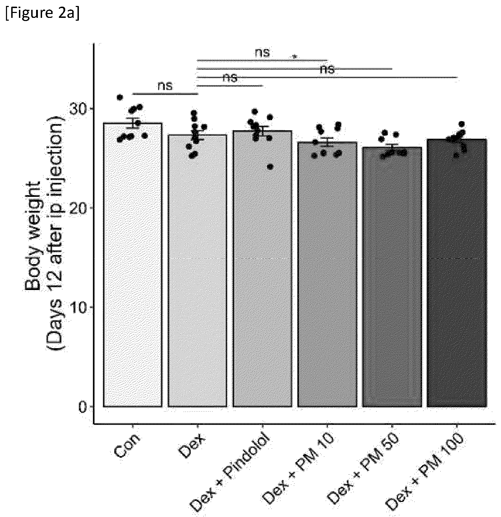

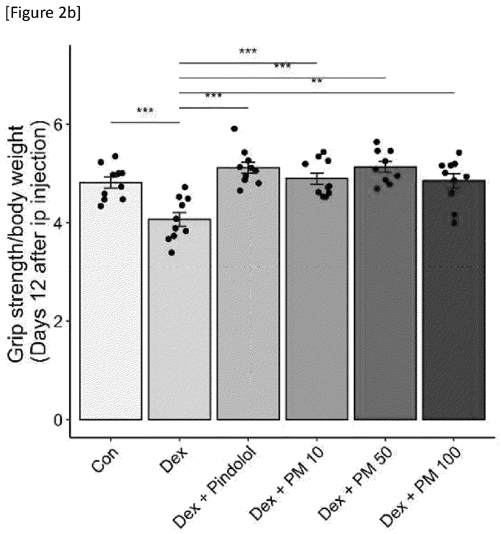

Figure 2a shows the results of body weight change by *Polygonum multiflorum* Thunberg extract. Figure 2b shows the results of grip strength improvement by *Polygonum multiflorum* Thunberg extract. (Con: control; Dex: Dexamethasone; PM 100: 100 mg/kg of *Polygonum multiflorum* Thunberg extract; PM 50: 50 mg/kg of *Polygonum multiflorum* Thunberg extract; PM 10: 10 mg/kg of *Polygonum multiflorum* Thunberg extract; *, p < 0.05; ** p < 0.01; ***, p < 0.001)

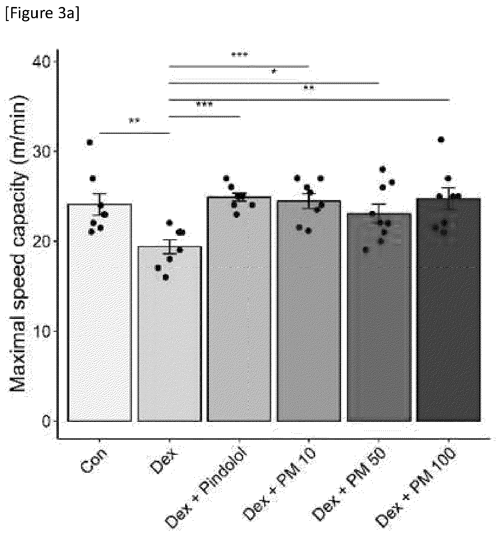

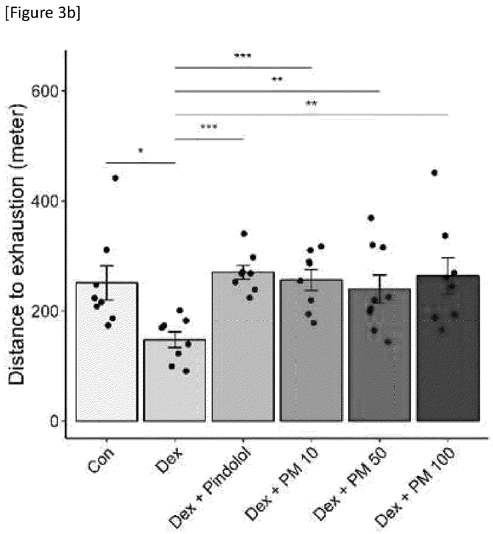

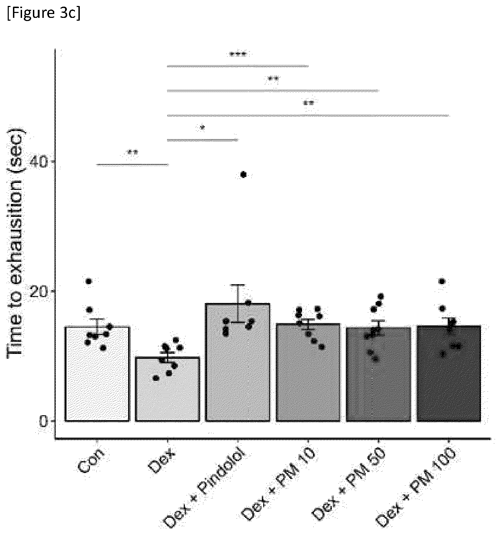

Figures 3a, 3b, and 3c show the results of the exercise performance improvement effect of *Polygonum multiflorum* Thunberg extract. Figure 3a: Maximal speed capacity, Figure 3b: Distance to exhaustion, Figure 3c: Time to exhaustion. (Con: control, Dex: Dexamethasone, PM 100: 100 mg/kg of *Polygonum multiflorum* Thunberg extract, PM 50: 50 mg/kg of *Polygonum multiflorum* Thunberg extract, PM 10: 10 mg/kg of *Polygonum multiflorum* Thunberg extract; *, *p <* 0.05; ** *p <* 0.01; ****, p <* 0.001))

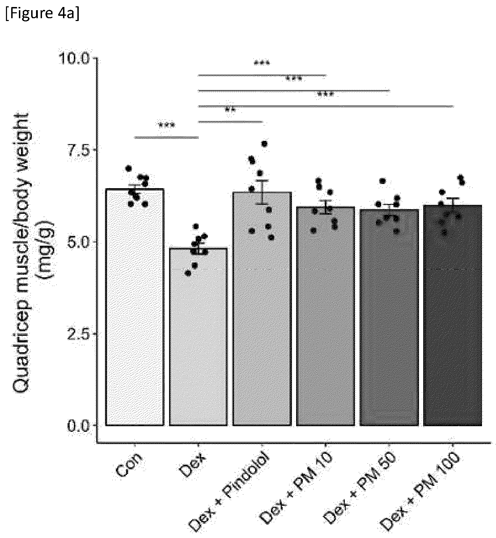

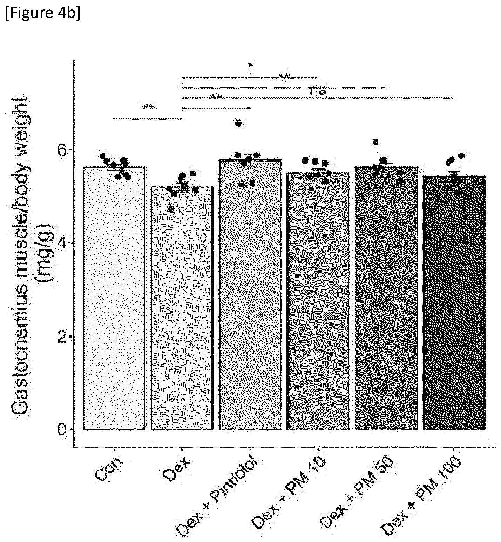

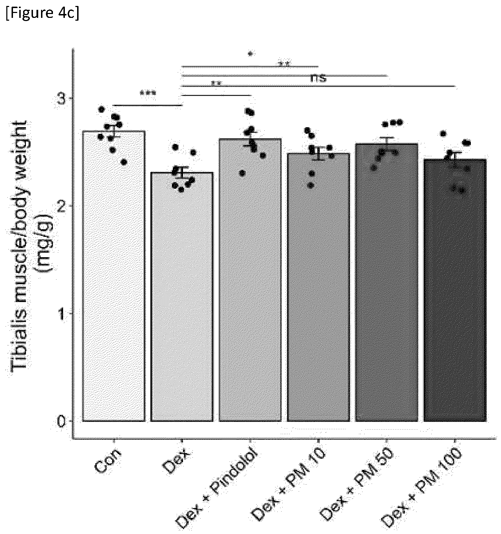

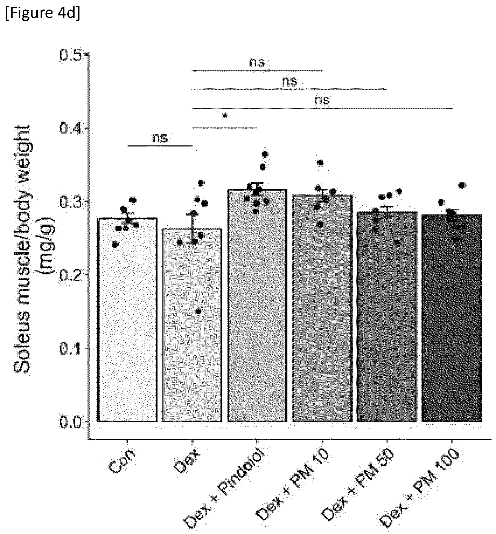

Figures 4a, 4b, 4c, and 4d show the results of the effects of the *Polygonum multiflorum* Thunberg extract in increasing muscle weight. Figure 4a: Quadricep muscle weight, Figure 4b: Gastrocnemius muscle weight, Figure 4c: Tibialis muscle weight, Figure 4d: Soleus muscle weight. (Con: control, Dex: Dexamethasone, PM 100: 100 mg/kg of *Polygonum multiflorum* Thunberg extract, PM 50: 50 mg/kg of *Polygonum multiflorum* Thunberg extract, PM 10: 10 mg/kg of *Polygonum multiflorum* Thunberg extract; *, *p <* 0.05; ** *p <* 0.01; ***, *p <* 0.001)

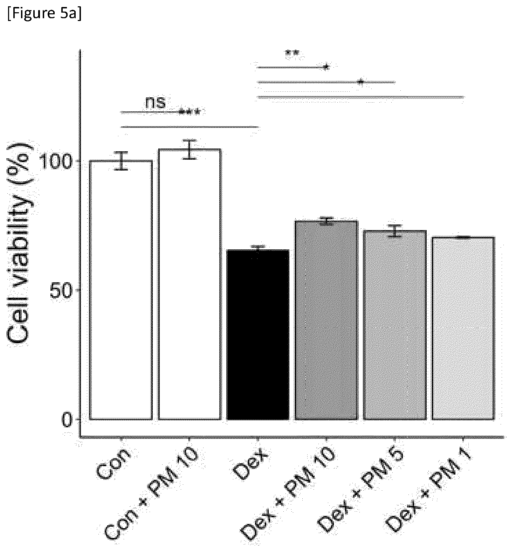

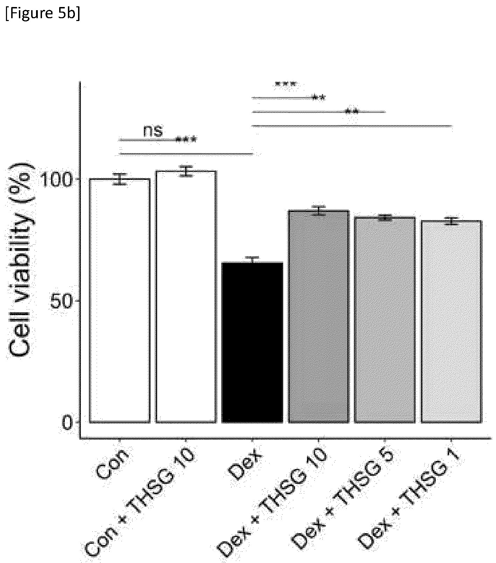

Figure 5a shows the results of the effects of *Polygonum multiflorum* Thunberg extract on cell viability. Figure 5b shows the results of the effects of THSG compounds on cell viability. (Con: control; Dex: Dexamethasone; PM 10: 10 ug/ml of *Polygonum multiflorum* Thunberg extract; PM 5: 5 ug/ml of *Polygonum multiflorum* Thunberg extract; PM 1: 1 ug/ml of *Polygonum multiflorum* Thunberg extract; THSG 10: 10 ug/ml of THSG; THSG 5: 5 ug/ml of THSG; THSG 1: 1 ug/ml of THSG; *, *p <* 0.05; ** *p <* 0.01; ***, *p <* 0.001)

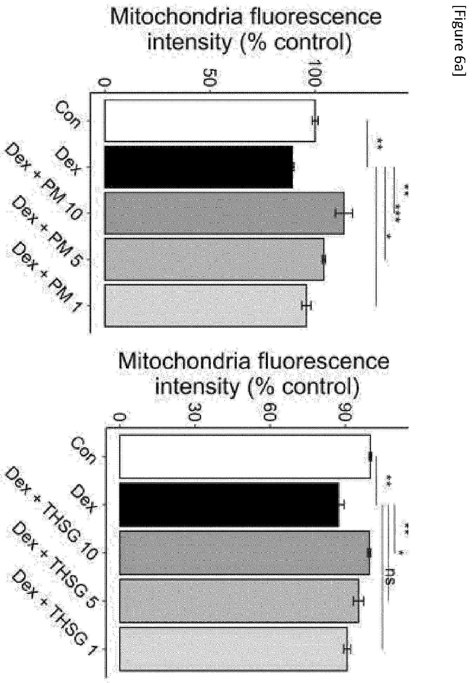

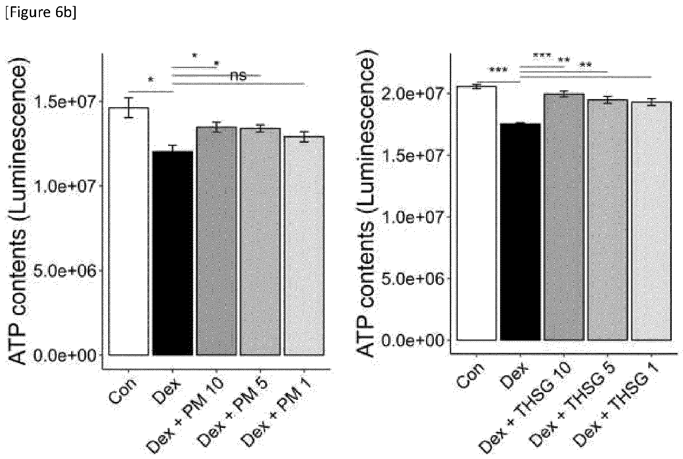

Figure 6a shows the results of improving mitochondrial function of *Polygonum multiflorum* Thunberg extract and THSG compounds. Figure 6b shows the results of increasing ATP production of *Polygonum multiflorum* Thunberg extract and THSG compounds. (Con: control, Dex: Dexamethasone, PM 10: 10 ug/ml of *Polygonum multiflorum* Thunberg extract, PM 5: 5 ug/ml of *Polygonum multiflorum* Thunberg extract, PM 1: 1 ug/ml of *Polygonum multiflorum* Thunberg extract, THSG 10: 10 ug/ml of THSG, THSG 5: 5 ug/ml of THSG, THSG 1: 1 ug/ml of THSG; *, *p <* 0.05; ** *p <* 0.01; ****, p <* 0.001)

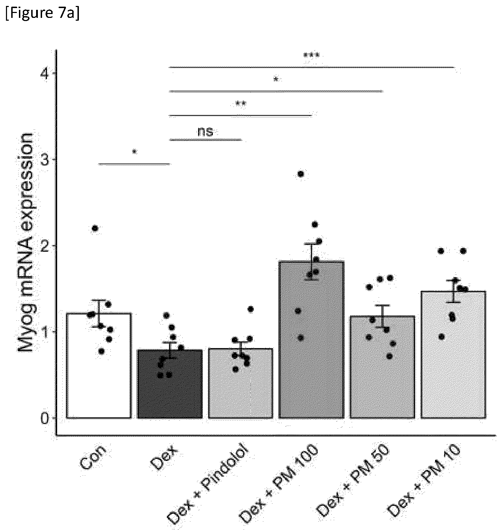

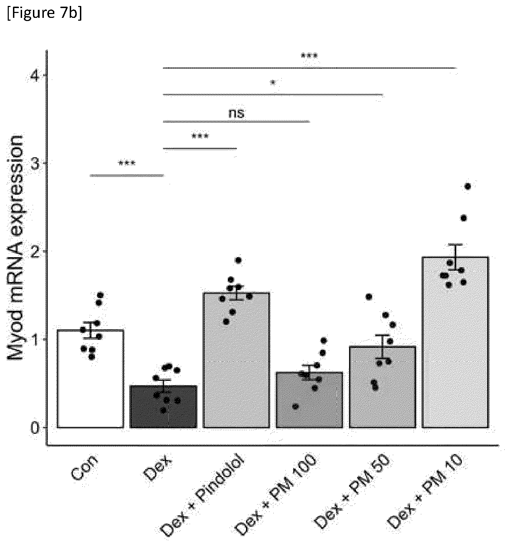

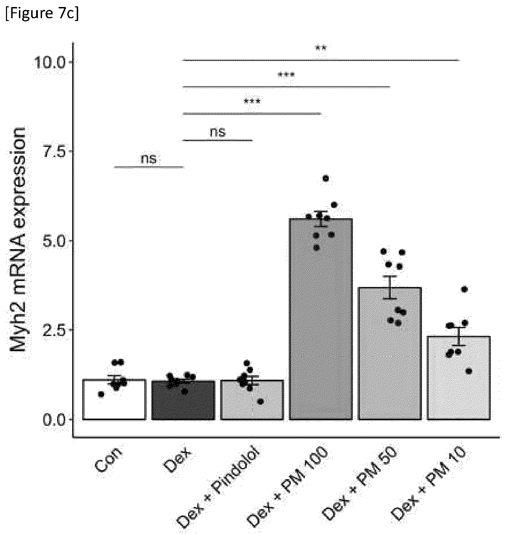

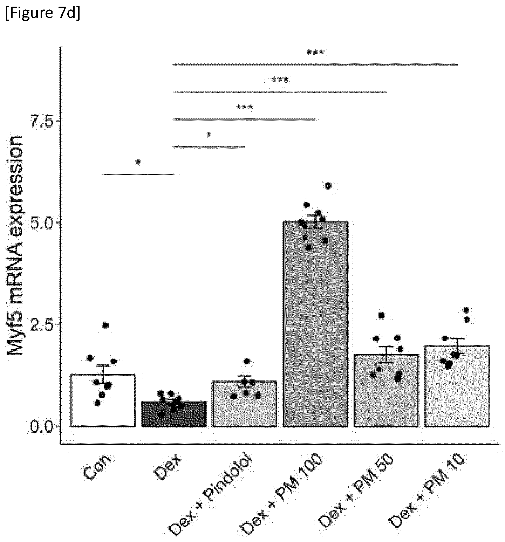

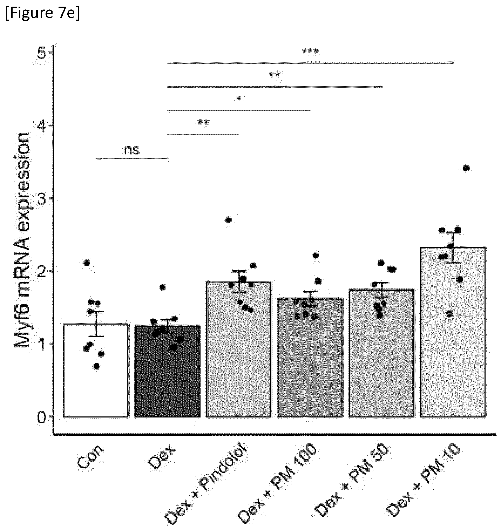

Figures 7a, 7b, 7c, 7d, and 7e show the results of the effects of *Polygonum multiflorum* Thunberg extract on enhancing the expression of muscle cell differentiation genes. Figure 7a: *Myog* mRNA expression, Figure 7b: *Myod* mRNA expression, Figure 7c: *Myh2* mRNA expression, Figure 7d: *Myf5* mRNA expression, Figure 7e: *Myf6* mRNA expression. (Con: control, Dex: Dexamethasone, PM 10: 10 ug/ml of *Polygonum multiflorum* Thunberg extract, PM 50: 50 ug/ml of *Polygonum multiflorum* Thunberg extract, PM 100: 100 ug/ml of *Polygonum multiflorum* Thunberg extract; *, *p <* 0.05; ** *p <* 0.01; ***, *p <* 0.001)

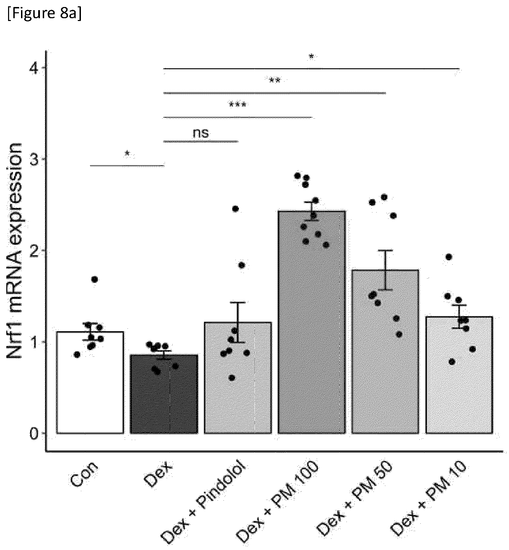

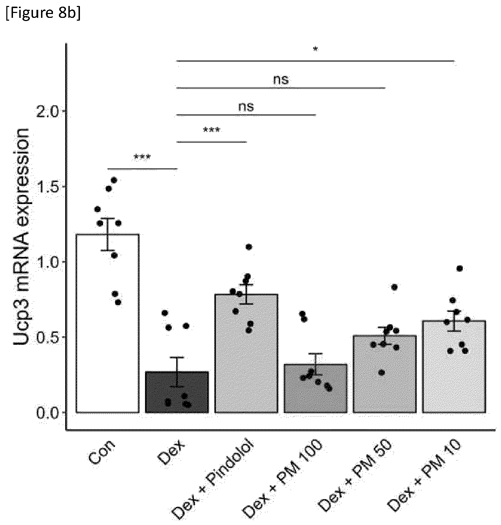

Figures 8a and 8b show the results of the effects of *Polygonum multiflorum* Thunberg extract in enhancing the expression of exercise performance-related genes. Figure 8a: *Nfr1* mRNA expression, Figure 8b: *Ucp3* mRNA expression. (Con: control, Dex: Dexamethasone, PM 10: 10 ug/ml of *Polygonum multiflorum* Thunberg extract, PM 50: 50 ug/ml of *Polygonum multiflorum* Thunberg extract, PM 100: 100 ug/ml of *Polygonum multiflorum* Thunberg extract; *, *p <* 0.05; ** *p <* 0.01; ***, *p <* 0.001)

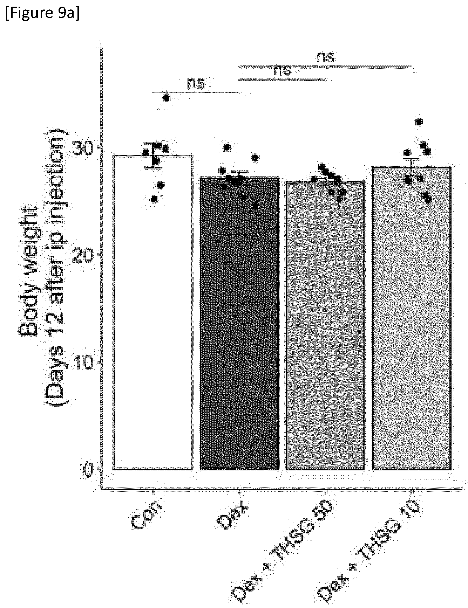

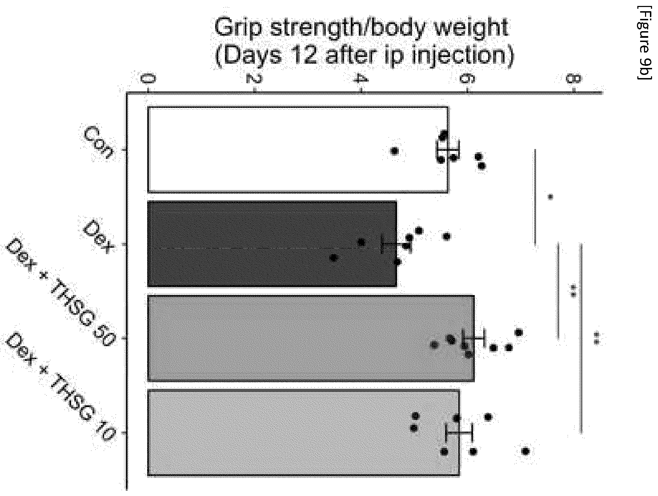

Figure 9a shows the results of body weight change by THSG compounds. Figure 9b shows the results of grip strength improvement by THSG compounds. (Con: control, Dex: Dexamethasone, THSG 50: THSG 50 mg/kg, THSG 10: THSG 10 mg/kg; *, *p <* 0.05; ** *p <* 0.01; ***, *p* < 0.001).

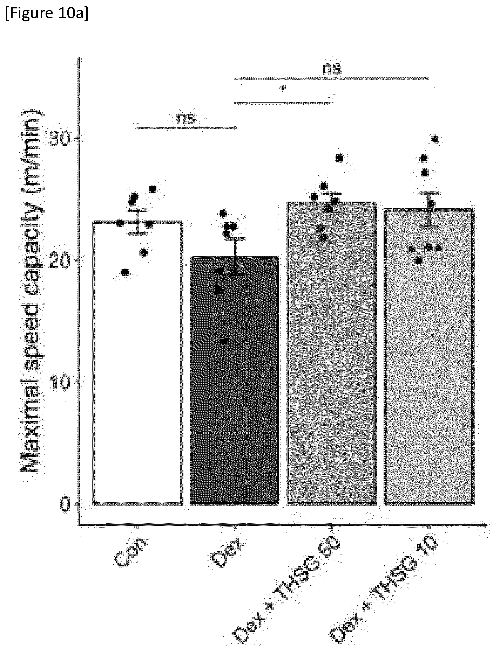

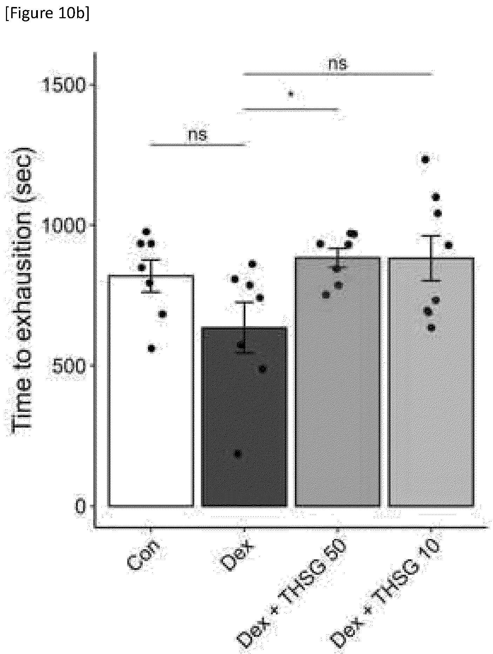

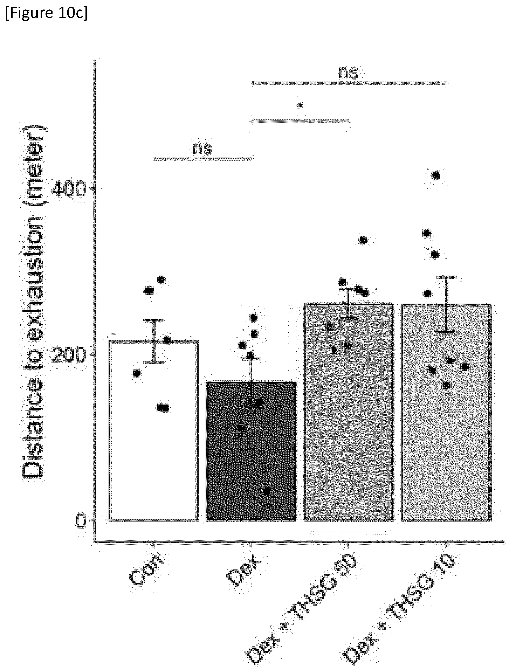

Figures 10a, 10b, and 10c show the results of the THSG compounds in improving exercise performance. Figure 10a: maximal speed capacity, Figure 10b: time to exhaustion, Figure 10c: distance to exhaustion (Con: control, Dex: dexamethasone, THSG 50: THSG 50 mg/kg, THSG 10: THSG 10 mg/kg; *, *p* < 0.05; ** *p* < 0.01; ***, *p* < 0.001).

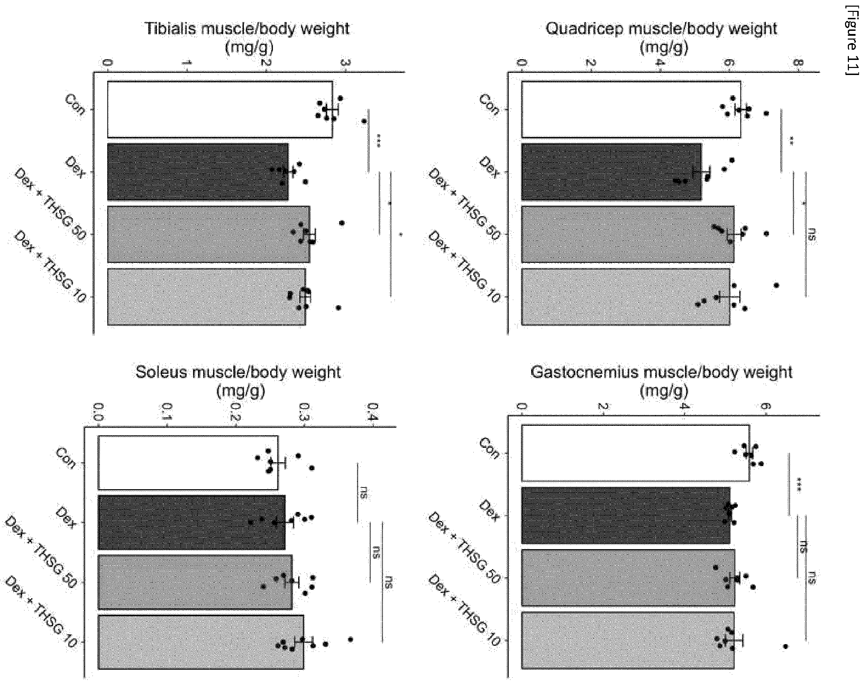

Figure 11 shows the effect of THSG compounds on increasing quadricep muscle weight, gastrocnemius muscle weight, tibialis muscle weight, and soleus muscle weight (Con: control; Dex: Dexamethasone; THSG 50: THSG 50 mg/kg; THSG 10: THSG 10 mg/kg; *, p < 0.05; ** *p <* 0.01; ***, *p* < 0.001).

### [Mode of the Invention]

The present inventors completed the present invention by experimentally confirming that the extract of *Polygonum multiflorum* Thunberg, a natural product with few or no side effects, is effective in increasing the expression of genes related to muscle cell differentiation and exercise performance, increasing muscle mass/weight, and improving exercise performance.

Hereinafter, the present invention will be described in detail.

The present invention provides a composition for enhancing exercise performance, increasing muscle mass, improving muscle function, or preventing, ameliorating, or treating muscular disease, comprising *Polygonum multiflorum* Thunberg extract as an active ingredient.

The present invention also provides a composition for enhancing exercise performance, increasing muscle mass, improving muscle function, or preventing, ameliorating, or treating muscular disease, comprising a compound represented by Formula 1 as an active ingredient below (2,3,5,4'-Tetrahydroxystilbene-2-O-β-D-glucoside, THSG):

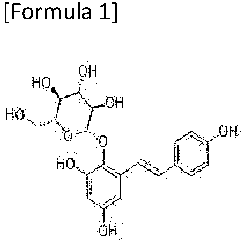

The composition may enhance muscle/grip strength.

The composition may increase ATP production

The composition may increase the expression of one or more genes selected from the group consisting of *Myod, Myog, Myh2, Myf5, Myf6, Nfr1,* and *Ucp3.*

The composition may improve exercise performance.

The composition may increase muscle mass/weight.

The present extract of *Polygonum multiflorum* Thunberg may comprise a compound represented by Formula 1.

The muscular disease may be one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

The composition may be a food composition, a health functional food composition, a pharmaceutical composition, or a quasi-drug composition.

As used herein, "preventing" means any act of delaying the onset of a muscular disease by administration of a composition of the invention, and "treating" and "ameliorating/ improving" means any act of ameliorating or beneficially altering the symptoms of a muscular disease by administration of a composition of the invention.

The present extract of *Polygonum multiflorum* Thunberg can be extracted using polar solvents and/or non-polar solvents.

The polar solvent may comprise one or more selected from the group consisting of (i) water, (ii) alcohol (preferably, methanol, ethanol, propanol, butanol, normal-propanol, isopropanol, normal-butanol, 1-pentanol, 2-butoxyethanol or ethylene glycol), (iii) acetic acid, (iv) dimethyl-formamide (DMFO), and (v) dimethyl sulfoxide (DMSO). The non-polar solvent may include at least one selected from the group consisting of acetone, acetonitrile, ethyl acetate, methyl acetate, fluoroalkanes, pentane, hexane, 2,2,4-trimethylpentane, decane, cyclohexane, cyclopentane, diisobutylene, 1-pentene, 1-chlorobutane, 1-chloropentane, o-xylene, diisopropyl ether, 2-chloropropane, toluene, 1-chloropropane, chlorobenzene, benzene, diethyl ether, diethyl sulfide, chloroform, dichloromethane, 1,2-dichloroethane, aniline, diethylamine, ether, carbon tetrachloride, and THF.

More specifically, the extract may be extracted using water, an organic solvent, or a mixture thereof as a solvent, and according to certain embodiments of the present invention, the extract of the present invention may be obtained by treating *Polygonum multiflorum* Thunberg with water.

The organic solvent may comprise one or more solvents selected from the group consisting of lower alcohols (methanol, ethanol, propanol, butanol, etc.) having a carbon number of 1 to 6, hexane, acetone, ethyl acetate, chloroform, and diethyl ether.

Furthermore, the extract of *Polygonum multiflorum* Thunberg may be prepared as a fraction according to methods conventionally used in the art, and the solvent used to fractionate the hydrolyzed extract may one or more selected from the group consisting of water, lower alcohols of C1 to C4, n-hexane, ethyl acetate, acetone, acetonitrile, butyl acetate, 1,3-butylene glycol, chloride, and mixtures which the acetate fraction is most preferred.

As used herein, the term "extract" has the meaning commonly understood in the art as a crude extract as described above, but broadly includes fractions obtained by further fractionation of the extract, i.e., the extract of *Polygonum multiflorum* Thunberg is not only obtained using the extraction solvent described above, but also includes fractions obtained by further purification. For example, fractions obtained by passing the extract through an ultrafiltration membrane having a certain molecular weight cut-off value, fractions obtained by additionally performing various purification methods such as separation by various chromatographies (designed for separation according to size, charge, hydrophobicity or affinity), etc. are also included in the natural product extract of the present invention.

The extract used in the present invention can be prepared in powder form by additional processes such as reduced pressure distillation and freeze drying or spray drying.

Specifically, the present extract can be extracted by adding water, an organic solvent, or a mixture thereof, to *Polygonum multiflorum* Thunberg in an amount of 5 times to 50 times the weight of *Polygonum multiflorum* Thunberg, more preferably in an amount of 10 times to 30 times the weight of *Polygonum multiflorum* Thunberg, but not limited to. The extraction temperature may be 10 °C to 150 °C, and is more preferably 15 °C to 120 °C, but is not limited thereto. The extraction time may be preferably from 1 hour to 20 hours, and more preferably from 2 hours to 8 hours, but is not limited thereto. The extraction method may be, but is not limited to, cold immersion, ultrasonic extraction, or reflux cooling extraction. The number of extraction times may be preferably from 1 to 5, and more preferably from 2 to 3 repeated extractions. Further, the extract may be diluted, concentrated, or diluted or concentrated and then purified and dried for use.

As used herein, the term "comprising as an active ingredient" means containing a sufficient amount to achieve the efficacy or activity of the *Polygonum multiflorum* Thunberg extract or THSG compound. The present invention also relates to a composition derived from the natural product, *Polygonum multiflorum* Thunberg, and the upper limit of the amount of *Polygonum multiflorum* Thunberg extract or THSG compound included in the composition of the invention may be selected by those skilled in the art within any suitable range.

### Pharmaceutical composition for enhancing exercise performance, increasing muscle mass, improving muscle function, or preventing or treating muscular disease comprising Polygonum multiflorum Thunberg extract or THSG compound as active ingredients

The composition of the present invention can be formulated as a pharmaceutical composition.

When the composition of the present invention is formulated as a pharmaceutical composition, the pharmaceutical composition of the present invention may comprise a pharmaceutically acceptable carrier.

According to a preferred embodiment of the present invention, the composition of the present invention may be a pharmaceutical composition comprising (a) a pharmaceutically effective amount of a *Polygonum multiflorum* Thunberg extract or THSG compound of the present invention as described above; and (b) a pharmaceutically acceptable carrier. As used herein, the term "pharmaceutically effective amount" means an amount sufficient to achieve the efficacy or activity of the *Polygonum multiflorum* Thunberg extract or THSG compound described above.

Pharmaceutically acceptable carriers are those customarily used in the formulation: lactose, dextrose, sucrose, sorbitol, mannitol, starch, acacia gum, calcium phosphate, alginate, gelatin, Pharmaceutically acceptable carriers are those customarily used in the formulation: lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oils, but not limited thereto. In addition to the above ingredients, the pharmaceutical composition of the present invention may further include lubricants, wetting agents, sweeteners, flavoring agents, emulsifiers, suspending agents, preservatives, etc. Suitable pharmaceutically acceptable carriers and formulations are described in detail in Remington's Pharmaceutical Sciences (19th ed., 1995).

The pharmaceutical composition of the present invention can be administered orally or parenterally.

Suitable dosages of the pharmaceutical composition of the present invention can be prescribed in a variety of ways, depending on factors such as the method of formulation, mode of administration, patient age, weight, sex, medical condition, food, time of administration, route of administration, rate of excretion, and response sensitivity. Typical dosages of the active ingredients in the pharmaceutical composition of the present invention are in the range of 0.001-100 mg/kg/day, preferably 0.01-35 mg/kg/day in adults. The dose may be administered once daily or may be divided into several doses. However, the above dosages do not limit the scope of the present invention.

The pharmaceutical composition of the present invention may be prepared in unit dose form or by formulation with pharmaceutically acceptable carriers and/or excipients, in accordance with methods readily practiced by a person of ordinary skill in the art to which the invention belongs. The formulation may be in the form of a solution, suspension, syrup, or emulsion in an oil or aqueous medium, or in the form of an excipient, acid, powder, granule, tablet, or capsule, and may further comprise a dispersant or stabilizing agent.

### Quasi-drug composition for the purpose of enhancing exercise performance, increasing muscle mass, improving muscle function, or preventing or ameliorating muscular disease comprising Polygonum multiflorum Thunberg extract or THSG compounds as active ingredients

The composition of the present invention can be provided as a quasi-drug composition.

The above-mentioned *Polygonum multiflorum* Thunberg extract or THSG compound may be added as is, or may be used together with ingredients such as other quasi-drugs, and may be used appropriately according to conventional methods. The mixing amount of the active ingredient can be appropriately determined depending on the purpose of use (prevention, health, or therapeutic treatment). The quasi-drug composition may be used in the manufacture of external preparations, patches, ointments, etc., but is not limited thereto.

### Food composition for enhancing exercise performance, increasing muscle mass, improving muscle function, or preventing or ameliorating muscular disease comprising Polygonum multiflorum Thunberg extract or THSG compound as an active ingredient

The composition of the present invention can be provided as a food composition or a health functional food composition.

When the composition comprising a *Polygonum multiflorum* Thunberg extract or THSG compound of the present invention as an active ingredient is manufactured as a food composition, it may include not only the extract or THSG compound of the present invention, but also ingredients that are customarily added in the manufacture of food products, for example, proteins, carbohydrates, fats, nutrients, seasonings and flavorings. Examples of the carbohydrates described above are monosaccharides, e.g., glucose, fructose, and the like; disaccharides, e.g., maltose, sucrose, oligosaccharides, and the like; and polysaccharides, e.g., dextrins, cyclodextrins, and the like, and sugar alcohols, e.g., xylitol, sorbitol, erythritol, and the like. As flavoring agents, natural flavoring agents [such as thaumatin, stevia extract (e.g., rebaudioside A, glycyrrhizin, etc.)] and synthetic flavoring agents (such as saccharin, aspartame, etc.) can be used. For example, if the food composition of the present invention is formulated as a beverage, it may additionally contain citric acid, liquid dextrose, sugar, glucose, acetic acid, malic acid, fruit juice, caterpillar extract, jujube extract, licorice extract, etc. in addition to the natural product extract of the present invention.

The formulation of the food composition or health functional food composition may be in the form of an acid, granule, pill, tablet, capsule, or capsule, as well as in the form of a conventional food or beverage.

Examples of food products to which the substance may be added include, without limitation, meat, sausages, bread, chocolate, candy, snacks, confectionery, pizza, ramen, other noodles, chewing gum, dairy products including ice cream, various soups, beverages, teas, drinks, alcoholic beverages and vitamin complexes, all of which are food products in the ordinary sense of the term.

In general, when manufacturing food or beverages, the extract or THSG compound of the present invention can be added in an amount of 15 parts by weight or less, preferably 10 parts by weight or less, based on 100 parts by weight of raw materials. However, in the case of long-term intake for the purpose of health and hygiene or health control, the amount may be below the above range, and the present invention may also be used in amounts above the above range, as the invention is safe in that it utilizes a natural product.

Hereinafter, the present invention will be described in more detail through examples. The purpose, features, and advantages of the present invention will be easily understood through the following examples. The present invention is not limited to the embodiments described herein and may be embodied in other forms. The embodiments introduced here are provided to enable the idea of the present invention to be sufficiently conveyed to those skilled in the art. Therefore, the present invention should not be limited by the following examples.

### [Preparation Example 1] Preparation of extract of Polygonum multiflorum Thunberg

*Polygonum multiflorum* Thunberg was purchased from Kyunghee Korean Medicine Co., Ltd. and cut into suitable size, and 50 g of *Polygonum multiflorum* Thunberg was added to the extraction vessel with 300 mL of water, 25% (v/v) ethanol, 50% (v/v) ethanol, 75% (v/v) ethanol, and 95% (v/v) ethanol, respectively, for 3 days, and the precipitate was removed using a centrifuge, and the impurities were filtered to obtain the filtrate. The solvent was then concentrated under reduced pressure and dried to give the extract.

### [Preparation Example 2] Preparation of fractions thereof

The water extract obtained according to Preparation Example 1 was subjected to solvent fractionation to obtain hexane (Hx), ethyl acetate (EA), butanol (BuOH), and water fractions. The preparation of each fraction was performed by adding distilled water to the water extract, suspending it, adding an equal amount of hexane, separating it into a hexane layer and a water layer, and filtering and concentrating under reduced pressure to obtain a hexane fraction (2.2 g). Then, the hexane fraction was removed, and an equal amount of ethyl acetate (EA) was added to the remaining water layer, and an ethyl acetate fraction (10.5 g) was obtained in the same manner as above. The ethyl acetate fraction was removed, and an equal amount of butanol (BuOH) was added to the remaining water layer, and a butanol fraction (25.3 g) and a water fraction (460 g) were obtained in the same manner as above.

**[Preparation Example 3] Isolation of compounds derived from *Polygonum multiflorum* Thunberg extract**

The ethyl acetate fraction obtained according to Preparation Example 2 was separated using a silica gel open column under the condition of Hx:EA=20:1-100% EA, and separated by molecular weight using a Sephadex open column under the condition of 50% methanol, and a compound represented by the following chemical formula (2,3,5,4'-Tetrahydroxystilbene-2-O-β-D-glucoside, THSG) was obtained using prep-MPLC (Fig. 1a):

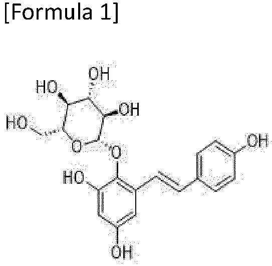

### [Example 1] Confirmation of changes in muscle strength of mice in which sarcopenia was induced by administration of Dexamethasone using Polygonum multiflorum Thunberg extract

In order to determine the effectiveness of treating or ameliorating sarcopenia with the *Polygonum multiflorum* Thunberg extract prepared according to the above Preparation Example, the inventors created a mouse model of sarcopenia and measured muscle strength using control mice and the sarcopenia mouse model.

The mice used to determine the efficacy of the extract of *Polygonum multiflorum* Thunberg were prepared as follows. As shown in Figure 1b, C57BL/6J (male, 8 weeks old) were from Dooyeol Biotech Co., Ltd. and acclimatized for 2 weeks, then divided into 6 groups (n=10 per group) according to body weight. The diets, doses, and methods of administration of the experimental groups were shown in Table 2 below. The test substance was prepared as a liquid suspension using 0.5% CMC, and the test substance was administered for 4 weeks. The vehicle control group was orally administered 0.5% CMC daily to compensate for the placebo effect of 0.5% CMC and the weight loss effect caused by the stress of administration. Two weeks after the administration of the test substance, the experimental group was administered Dexamethasone intraperitoneally for two weeks to induce sarcopenia, and the normal control group was administered saline intraperitoneally. All mice were fed the same AIN76 diet (Research Diets) and the test substances were administered for an additional 2 weeks, for a total of 4 weeks. The dark : light cycle was maintained at a 12 hr : 12 hr interval and water was provided ad libitum. As shown in Figures 2a and 2b, the results showed that the muscle strength decreased over time in the Dexamethasone (Dex)-treated group and increased in the group of mice treated with the positive control Pindolol and *Polygonum multiflorum* Thunberg extract compared to the group treated with only Dexamethasone. [Dex: Dexamethasone: sarcopenia-inducing substance (25 mg/kg), PM: *Polygonum multiflorum* Thunberg extract (10, 50, or 100 mg/kg)]

**[Table 1]**

| Test group | Feed | Sarcopenia-inducing substance | Orally administered substance | Dosage and method of administrati on | Administration period |
|---|---|---|---|---|---|
| Con | AIN76 diet | Saline | Vehicle | | After 2 weeks of oral administration of test substance, 2 weeks of administration of test substance and inducing substance |
| Dex | | Dexamethasone (25 mg/kg, once per day, 2 weeks) | Vehicle | | |
| Dex + Pindolol | | | Pindolol | 5 mg/kg, once per day | |
| Dex + PM 100 | | | Extract of *Polygonum* | 100 mg/kg, once per day | |
| Dex + PM 50 | | | | 50 mg/kg, once per day | |
| Dex + PM 10 | | | | 10 mg/kg, Once per day | |

### [Example 2] Confirmation of the efficacy of improving exercise performance in mice in which sarcopenia was induced by Dexamethasone administration using Polygonum multiflorum Thunberg extract

After a total of 4 weeks of treatment with the *Polygonum multiflorum* Thunberg extract in Example 1 above, exercise performance was evaluated for all treatment groups. Specifically, maximum running speed, distance to exhaustion, and time to exhaustion were evaluated, and the results are shown in Figures 3a, 3b, and 3c. As shown in Figures 3a, 3b, and 3c, it was confirmed that exercise performance significantly increased in the group administered with the extract of *Polygonum multiflorum* Thunberg.

### [Example 3] Confirmation of weight gain by muscle type in mice in which sarcopenia was induced by Dexamethasone administration using Polygonum multiflorum Thunberg extract

After the completion of the administration of the extract of *Polygonum multiflorum* Thunberg in [Example 1] for a total of 4 weeks, the weight of each type of muscle tissue in all treatment groups was confirmed.

As a result, as shown in Figures 4a, 4b, 4c, and 4d, compared to the control group administered saline, in the dexamethasone administered group, the weights of the quadricep muscles, gastrocnemius muscles, tibialis muscles, and soleus muscles were confirmed to decrease, but when the extract of *Polygonum multiflorum* Thunberg was administered, the weights of these muscles were confirmed to significantly increase.

### [Example 4] Confirmation of the efficacy of Polygonum multiflorum Thunberg extract and THSG compound on improving myotube cell survival rate in Dexamethasone-induced muscle atrophy in C2C12 myotube cells

Cell culture and differentiation of myoblasts: Myoblasts derived from C2C12 mice (CRL1772; American type cell collection, USA) were cultured in DMEM (Dulbecco's Modified Eagle's Medium) medium containing 10 (v/v)% FBS, and after reaching 90% confluence, they were transferred to DMEM medium containing 2 (v/v)% horse serum and cultured for 7 days to differentiate into myotubes.

After differentiating C2C12 myoblasts into myotubes, myoatrophy was induced by treating with 100 µM dexamethasone for 24 hours, and the myoatrophy alleviation effect was confirmed by treating with 10, 5, and 1 ug/ml concentrations of *Polygonum multiflorum* Thunberg extract (PM) and THSG compound. As a result, as shown in Fig. 5, it was confirmed that the survival rate of myotube cells decreased by dexamethasone treatment was restored in the *Polygonum multiflorum* Thunberg extract and THSG treatment groups.

### [Example 5] Confirmation of the efficacy of Polygonum multiflorum Thunberg extract and THSG compound in enhancing muscle function (increased ATP production)

As in Example 4, C2C12 myoblasts were differentiated into myotubes, and then oxidative stress or mitochondrial dysfunction was induced by treating with 10 µM dexamethasone for 24 hours. Then, the functional improvement efficacy was confirmed by treating with the *Polygonum multiflorum* Thunberg extract and THSG at concentrations of 10, 5, and 1 ug/ml, respectively. It was confirmed that mitochondrial dysfunction induced by dexamethasone treatment was improved by the *Polygonum multiflorum* Thunberg extract and THSG treatment. In addition, the muscle function improvement (increased ATP production) effect by the *Polygonum multiflorum* Thunberg extract and THSG treatment was confirmed (Figures 6a and 6b).

### [Example 6] Confirmation of the efficacy of Polygonum multiflorum Thunberg extract in enhancing myotube differentiation and exercise performance-related gene expression in Dexamethasone-induced muscle atrophy in C2C12 myotube cells

C2C12 mouse-derived myotubes treated with 10 µM dexamethasone were washed twice with PBS. Total RNA was extracted according to the user-recommended protocol of the RNA extraction kit (GeneAll RNA isolation kit, Seoul, South Korea). cDNA was synthesized from the extracted RNA and real-time quantitative polymerase chain reaction (RT-qPCR) was performed. PCR analysis was performed according to the user-recommended cycling conditions of the analytical instrument (Applied Biosystems 7900 HT thermal cycler, Applied Biosystems). To confirm the expression level of each mRNA, the fold change value compared to the control group was calculated using beta-actin as an endogenous control and the relative expression level was compared. The primer sequences are as follows:

**[Table 2]**

| Gene | Primer type | Sequence | Sequence number |
|---|---|---|---|
| *Myod* | forward | CAT AGA CTT GAC AGG CCC CG | SEQ ID NO: 1 |
| *Myod* | reverse | CGG GTC CAG GTC CTC AAA AA | SEQ ID NO: 2 |
| *Myog* | forward | AGC TAT CCG GTT CCA AAG CC | SEQ ID NO: 3 |
| *Myog* | reverse | GCA CAG GAG ACC TTG GTC AG | SEQ ID NO: 4 |
| *Myh2* | forward | AGC GAA GAG TAA GGC TGT CC | SEQ ID NO: 5 |
| *Myh2* | reverse | AGG CGC ATG ACC AAA GGT T | SEQ ID NO: 6 |
| *Myf5* | forward | TCC AGG TAT TCC CAC CTG CT | SEQ ID NO: 7 |
| *Myf5* | reverse | TCA GCT TTG TGT GCT CCG AA | SEQ ID NO: 8 |
| *Myf6* | forward | ACA GAT CGT CGG AAA GCA GC | SEQ ID NO: 9 |
| *Myf6* | reverse | CAC TCC GCA GAA TCT CCA CC | SEQ ID NO: 10 |
| *Nfr1* | forward | CCC GTG TTC CTT TGT GGT GA | SEQ ID NO: 11 |
| *Nfr1* | reverse | ATT CCA TGC TCT GCT GCT GG | SEQ ID NO: 12 |
| *Ucp3* | forward | GTT TTG CGG ACC TCC TCA CT | SEQ ID NO: 13 |
| *Ucp3* | reverse | CTC TGT GCG CAC CAT AGT CA | SEQ ID NO: 14 |

As shown in Figures 7a~7e and 8a~8b, we confirmed that the expression of genes related to muscle cell differentiation (*Myod, Myog, Myh2, Myf5,* and *Myf6*) that were reduced by dexamethasone treatment was increased by treatment with the extract of *Polygonum multiflorum* Thunberg, and the expression of genes related to exercise performance (*Nfr1* and *Ucp3)* was increased by treatment with the extract of *Polygonum multiflorum* Thunberg.

### [Example 7] Confirmation of the efficacy of THSG compound to change in muscle strength in mice in which sarcopenia was induced by administration of Dexamethasone

To determine the effectiveness of treating or ameliorating sarcopenia with a THSG compound prepared according to the above Preparation Example, the inventors created a sarcopenia mouse model as in Example 1, and measured muscle strength using control mice and a sarcopenia mouse model.

As shown in Figures 9a and 9b, it was found that the muscle strength decreased in the Dexamethasone (Dex)-treated group over time, and the muscle strength decreased by Dexamethasone-treatment was increased again in the THSG compound-treated mouse group [Dex: Dexamethasone: Sarcopenia-inducing substance (25 mg/kg), THSG: 2,3,5,4'-Tetrahydroxystilbene-2-O-β-D-glucoside (10 or 50 mg/kg)]

**[Table 3]**

| Test group | Feed | Sarcopenia-inducing substance | Orally administered substance | Dosage and method of administratio n | Administration period |
|---|---|---|---|---|---|
| Con | AIN76 diet | Saline | Vehicle | - | After 2 weeks of oral administration of test substance, 2 weeks of administration of test substance and inducing substance (Total 4 weeks) |
| Dex | | Dexamethasone (25 mg/kg, once a day, 2 weeks) | Vehicle | - | |
| Dex + THSG 50 | | | THSG | 50 mg/kg, once a day | |
| Dex + THSG 10 | | | | 10 mg/kg, once a day | |

### [Example 8] Confirming the efficacy of improving exercise performance in mice in which sarcopenia was induced by Dexamethasone administration using THSG compound

After the completion of the administration in [Example 7] for a total of 4 weeks, exercise performance was evaluated for all administration groups. Specifically, maximum running speed, distance run to exhaustion, and time taken to exhaustion were evaluated, and the results were shown in Figures 10a, 10b, and 10c. As shown in Figures 10a, 10b, and 10c, it was confirmed that exercise performance significantly increased in the group administered the THSG compound.

### [Example 9] Confirmation of weight increase by muscle type in mice in which sarcopenia was induced by administration of Dexamethasone using THSG compound

After the completion of the administration in [Example 7] for a total of 4 weeks, the weight of each type of muscle tissue was determined in all treatment groups.

As a result, as shown in Figure 11, compared to the control group administered saline, in the dexamethasone administered group, the weights of the quadricep muscles, gastrocnemius muscles, tibialis muscles, and soleus muscles were confirmed to decrease, but when the THSG compound was administered, the weights of these muscles were confirmed to significantly increase.

## Claims

1. A food composition for use in enhancing exercise performance, increasing muscle mass, improving muscle function, or preventing or ameliorating muscular disease, comprising *Polygonum multiflorum* Thunberg extract or a compound represented by Formula 1 as an active ingredient below as an active ingredient:

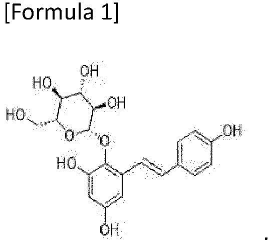

2. The food composition for use of claim 1, wherein the composition increases muscle strength.

3. The food composition for use of claim 1, wherein the composition increases ATP production.

4. The food composition for use of claim 1, wherein the composition increases the expression of one or more genes selected from the group consisting of *Myod, Myog, Myh2, Myf5, Myf6, Nfr1,* and *Ucp3.*

5. The food composition for use of claim 1, wherein the composition is extracted with a solvent selected from the group consisting of water, organic solvents, and mixtures thereof.

6. The food composition for use of claim 5, wherein the organic solvent is one or more selected from the group consisting of lower alcohols of carbon number 1 to 6, hexane, acetone, ethyl acetate, and diethyl ether.

7. The food composition for use of claim 1, wherein the *Polygonum multiflorum* thunberg extract comprises a compound represented by Formula 1:

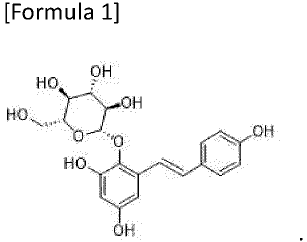

8. The food composition for use of claim 1, wherein the muscular disease is one or more diseases selected from the group consisting of sarcopenia, muscular atrophy, myasthenia, muscular dystrophy, sarcopenia, hypotonia, muscular weakness, muscular dystrophy, atony, amyotrophic lateral sclerosis, and inflammatory myopathy.

9. A quasi-drug composition for use in enhancing exercise performance, increasing muscle mass, improving muscle function, or preventing or ameliorating muscular disease, comprising *Polygonum multiflorum* Thunberg extract or a compound represented by Formula 1 as an active ingredient below as an active ingredient:

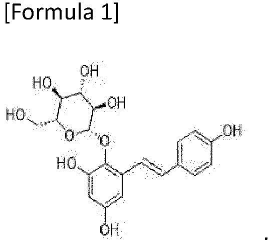

10. A pharmaceutical composition for use in enhancing exercise performance, increasing muscle mass, improving muscle function, or preventing or treating a muscle disease comprising *Polygonum multiflorum* Thunberg extract or a compound represented by Formula 1 as an active ingredient below as an active ingredient:
